(19) Europäisches Patentamt / European Patent Office / Office européen des brevets

(11) **EP 2 554 730 A1**

(12) **EUROPEAN PATENT APPLICATION**
published in accordance with Art. 153(4) EPC

(43) Date of publication:
**06.02.2013 Bulletin 2013/06**

(21) Application number: **11762592.1**

(22) Date of filing: **10.03.2011**

(51) Int Cl.:
*D04H 1/70* (2012.01)   *A61F 13/49* (2006.01)
*A61F 13/511* (2006.01)   *B32B 5/26* (2006.01)
*D04H 1/54* (2012.01)

(86) International application number:
**PCT/JP2011/056321**

(87) International publication number:
**WO 2011/122355 (06.10.2011 Gazette 2011/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.03.2010 JP 2010075824**

(71) Applicant: **Unicharm Corporation**
**Ehime 799-0111 (JP)**

(72) Inventors:
• **YAMAGUCHI, Masashi**
  **Kanonji-shi**
  **Kagawa 769-1602 (JP)**
• **OBA, Toru**
  **Kanonji-shi**
  **Kagawa 769-1602 (JP)**

(74) Representative: **Vaughan, Jennifer Ann**
  **Saunders & Dolleymore LLP**
  **9 Rickmansworth Road**
  **GB-Watford, Herts. WD18 0JU (GB)**

(54) **SHEET OF NONWOVEN FABRIC**

(57)   Provided is a sheet of nonwoven fabric for use in absorbent articles such as disposal diapers, sanitary napkins, wipes, etc., the sheet of nonwoven fabric being suitable for holding high-viscosity excreta such as soft feces. The sheet of nonwoven fabric has, formed in a first surface thereof, a plurality of ridges and grooves which extend in the machine direction. The sheet comprises a first fibrous layer and a second fibrous layer, the first fibrous layer being located on the first-surface side and the second fibrous layer being located on the second-surface side, which is on the reverse side from the first surface. The second fibrous layer comprises fibers having the ability to be crimped, and the first fibrous layer comprises heat-fusible fibers which are not crimped at the crimping initiation temperature of the fibers having the ability to be crimped. In a plan view from the first-surface side, the peripheries of the ridges have a shape which is composed of repeated meanders.

## Fig.13

EP 2 554 730 A1

**Description**

TECHNICAL FIELD

The present invention relates to a non-woven fabric sheet for absorbent articles such as disposable diapers, sanitary napkins or cleansing wipes, a production process thereof, and an absorbent article using the same.

BACKGROUND ART

Japanese Patent No. 3617637 discloses a surface sheet for an absorbent article that has surface properties that cause little frictional irritation of the skin, has a favorable feel on the skin and is resistant to the occurrence of skin problems such as itching or rash despite being capable of maintaining a macro surface structure that particularly enables highly viscous waste matter among urine (soft stool) and menstrual blood containing irritating substances to be rapidly absorbed without remaining on the surface. This surface sheet for an absorbent article has surface irregularities formed on the side that contacts the skin when wearing, and when wearing an absorbent article that uses this surface sheet, the surface irregularities elastically deform to follow the shape and movement of the body, and the recesses of the surface irregularities incorporate highly viscous waste matter and cause the highly viscous waste matter to separate from the body.

SUMMARY OF THE INVENTION

Problems to be Solved by the Invention

However, in the case of the example of the prior art described in Japanese Patent No. 3617637, a sheet for forming surface irregularities in which surface irregularities are pleated to form a large number of pleats formed mutually in parallel therein is formed by joining to a base sheet between the pleats to incorporate high viscous liquids. In addition, as is described in paragraph [0030] of Japanese Patent No. 3617637, a material used as a surface sheet in ordinary absorbent articles can be used without any particular limitations for the material used to form the sheet for forming surface irregularities in a surface sheet. In this case, the material used to form the sheet for forming surface irregularities in a surface sheet is produced by overlapping fibers mainly arranged in parallel with the machine direction in the direction of thickness. Although soft stool adhered to the skin is wiped off using a baby wipe or tissue when changing the diapers of newborns, from the viewpoints of reducing the bother of wiping off and cost advantages resulting from reducing the number of wipes or tissues used, there are many cases in which, when having opened up the diaper during changing, after preliminarily wiping using an unsoiled portion of the surface sheet of the diaper (such as the front) or a flap or back sheet, the skin is then cleanly wiped with a baby wipe or tissue. In such cases, the function of the surface sheet, flap or back sheet is required to have the conventional functions of absorption or covering by demonstrating the function of an outer member, but also a function that allows stool adhered to the skin to be wiped off. Since protrusions extend roughly linearly in the prior art, in the case of attempting to further increase surface area from the viewpoint of increasing the stool entrapment capacity per unit area, it is necessary to further increase the height of the protrusions. However, in the case of attempting to further increase the height of the protrusions, an awareness of the absorbent article being used on the skin ends up increasing. In addition, in the case of the structure of the prior art in which fibers, in which protrusions are mainly arranged in parallel in the machine direction, overlap in the direction of thickness, there is still room for improvement from the viewpoints of forming gaps between fibers capable of entrapping stool as well as maintaining gaps between fibers when compressed.

Means for Solving the Problems

In a first aspect thereof, the subject invention is a non-woven fabric sheet in which a plurality of ridges and grooves are formed on a first surface thereof extending in the machine direction, wherein the non-woven fabric sheet comprises a first fibrous layer on a first surface side and a second fibrous layer on a second surface side opposite from the first surface side, the second fibrous layer comprises latent crimpable fibers, the first fibrous layer comprises heat-fusible fibers that do not demonstrate crimping at the crimping starting temperature of the latent crimpable fibers, and when the non-woven fabric sheet is viewed from overhead from first surface side, the outer edges of the ridges have a repeated meandering shape.

Preferably, sites where the height of the ridges is relatively high over the direction in which the ridges extend and sites where it is relatively low are formed in the ridges.

Preferably, the ridges have an enhanced three-dimensional random orientation as a result of at least a portion of the fibers therein generating curvature or bending.

In a second aspect thereof, the subject invention is a process for producing a non-woven fabric sheet, comprising:

a) a step for opening a fiber assembly containing latent crimpable fibers by passing through a carding machine to form a web containing latent crimpable fibers,

b) a step for opening a fiber assembly containing heat-fusible fibers that do not demonstrate crimping at the crimping starting temperature of the latent crimpable fibers by passing through a carding machine to form a web containing heat-fusible fibers,

c) a step for superimposing the web containing latent crimpable fibers and the web containing heat-fusible fibers to form a laminated web,

d) a step for realigning the fibers so that ridges and grooves are formed in the laminated web, and portions in which the degree of fiber entanglement in the direction in which the ridges are continuous is relatively high and portions in which it is relatively low are alternately formed,

f) a step for heating the web in which fibers have been realigned to cause the latent crimpable fibers to demonstrate crimping, and

g) a step for heating the web in which crimping has been demonstrated by the latent crimpable fibers to fuse the heat-fusible fibers at sites where they mutually intersect.

Preferably, step d is a step in which the laminated web is transported by placing on a support, in which liquid passage portions and liquid blocking portions extending in parallel in a cross-machine direction are arranged by alternately repeating in a machine direction, while forming a plurality of ridges and grooves extending in parallel in the machine direction by spraying a liquid from a plurality of nozzles arranged in a row in the cross-machine direction onto the surface of the web side of the laminated web that contains the heat-fusible fibers.

Preferably, step f is a step in which the web is heated to a temperature at which the latent crimpable fibers demonstrate crimping and which is lower than the fusing temperature of the heat-fusible fibers.

Preferably, step f is carried out in a state in which there is little resistance to force that attempts to shrink the latent crimpable fibers in the machine direction as a result of crimping.

Preferably, the transport speed of step f is slower than that of the previous step.

Preferably, a floating dryer is used in step f.

Preferably, the process comprises e) a step for transporting the laminated web obtained in step d to step f between step d and step f.

Preferably, the process comprises h) a step for cooling the web obtained in step g after step g.

In a third aspect thereof, the subject invention is an absorbent article containing the non-woven fabric sheet.

Effects of the Invention

The non-woven fabric sheet of the present invention is able to efficiently entrap highly viscous waste matter in the manner of soft stool.

When the first surface side is viewed from overhead, the surface area per unit area can be increased and opportunities for contacting the target highly viscous waste matter can be increased by having the outer edges of the ridges extending in the machine direction while repeatedly meandering.

In addition, since the ridges and grooves are continuous, sheet rigidity in a fixed direction is enhanced and the formed entrapping spaces are resistant to crushing.

In addition, in the case in which sites where the height of the ridges is relatively high over the machine direction and sites where it is relatively low are alternately formed, the actual surface area per unit projected area is further increased, and opportunities for contacting the target highly viscous waste matter can be increased.

In addition, in the case three-dimensional, random orientation is enhanced in the ridges as a result of a portion of the fibers generating curvature or bending, the formation of gaps between fibers capable of entrapping stool and the maintaining of gaps between fibers when compressed are improved.

BRIEF DESCRIPTION OF THE DRAWINGS

FIG. 1 is a schematic diagram showing an enlarged overhead view of an embodiment of the non-woven fabric sheet of the present invention.

FIG. 2 is a schematic diagram of an enlarged overhead view of another embodiment of the non-woven fabric sheet of the present invention.

FIG. 3 is a schematic diagram of a cross-sectional view taken along line X-X' in FIG. 1.

FIG. 4 is a schematic diagram of a cross-sectional view taken along line Y-Y' in FIG. 1.

FIG. 5 is a drawing showing an example of the production steps of a non-woven fabric sheet of the present invention.

FIG. 6 is a drawing showing an example of the arrangement of nozzles used in step d.

FIG. 7 is a drawing showing an example of multiple rows of nozzles used in step d.

FIG. 8 is a drawing showing an example of a molding plate used in step d.

FIG. 9 is a schematic diagram for explaining a process by which ridges and grooves are formed.

FIG. 10 is a drawing showing a floating dryer able to be used in step f.

FIG. 11 is a drawing for explaining a method for measuring the amount of entrapped stool.

FIG. 12 is a drawing for explaining a method for measuring stool diffusion area.

FIG. 13 is an overhead perspective photograph of a first surface side of a non-woven fabric sheet of Example 3 as viewed from a machine direction.

FIG. 14 is an overhead perspective photograph of a first surface side of a non-woven fabric sheet of Example 3 as viewed from a cross-machine direction.

FIG. 15 is an overhead photograph of a non-woven fabric sheet of Example 3.

FIG. 16 is a photomicrograph of a cross-section in the cross-machine direction of a non-woven fabric sheet of Example 3.

FIG. 17 is a photomicrograph of a cross-section parallel to a machine direction that passes through the tops of the ridges of a non-woven fabric sheet of Example 3.

FIG. 18 is an overhead photograph of a non-woven fabric sheet of Example 1.

FIG. 19 is a photomicrograph of a cross-section in the cross-machine direction of a non-woven fabric sheet of Example 1.

FIG. 20 is a photomicrograph of a cross-section parallel to a machine direction that passes through the tops of the ridges of a non-woven fabric sheet of Example 1.

FIG. 21 is an overhead photograph of a non-woven fabric sheet of Comparative Example 1.

FIG. 22 is a photomicrograph of a cross-section in the cross-machine direction of a non-woven fabric sheet of Comparative Example 1.

FIG. 23 is a photomicrograph of a cross-section parallel to a machine direction that passes through the tops of the ridges of a non-woven fabric sheet of Comparative Example 1.

FIG. 24 is photograph showing the fiber orientation of ridges of a non-woven fabric sheet of Example 3.

FIG. 25 is a photograph showing the fiber orientation of ridges of a non-woven fabric sheet of Comparative Example 1.

EMBODIMENTS OF THE INVENTION

The following provides an explanation of the present invention based on preferred embodiments thereof with reference to the drawings.

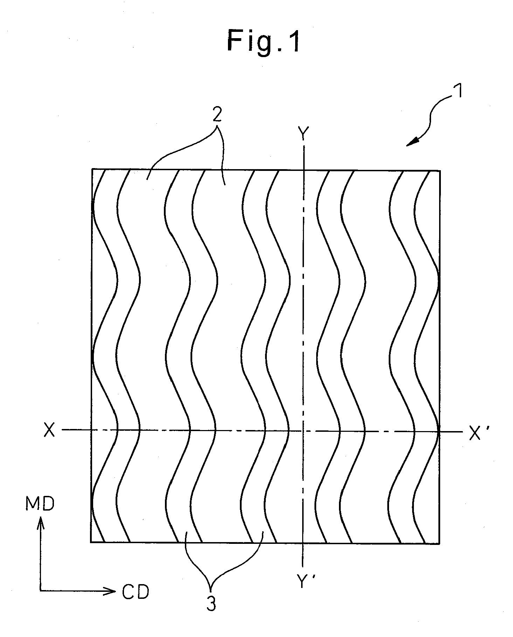

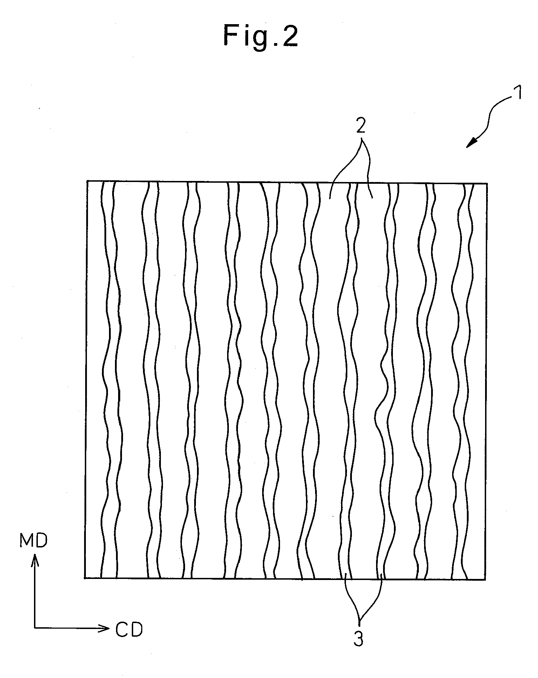

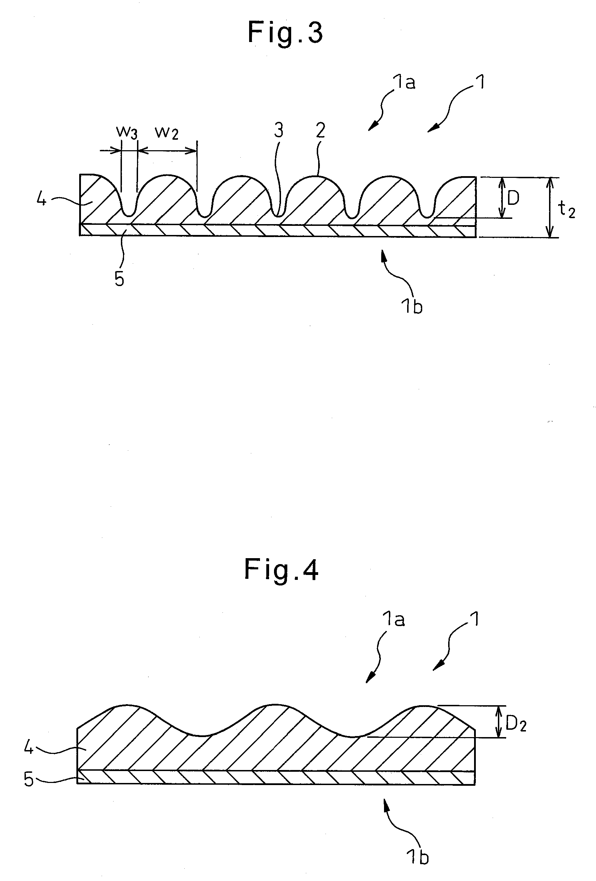

FIG. 1 shows a schematic diagram of an enlarged overhead view of a first embodiment of the non-woven fabric sheet of the present invention. FIG. 2 shows a schematic diagram of an enlarged overhead view of another embodiment of the non-woven fabric sheet of the present invention. FIG. 3 is a schematic diagram of a cross-sectional view taken along line X-X' in FIG. 1. FIG. 4 is a schematic diagram of a cross-sectional view taken along line Y-Y' in FIG. 1.

A non-woven fabric sheet 1 has a first surface 1a and a second surface 1b in opposition thereto.

The non-woven fabric sheet 1 has a large number of ridges 2 and grooves 3 extending in machine direction (to also be abbreviated as "MD") on the side of the first surface 1a. The ridges 2 and the grooves 3 are alternately arranged over a cross-machine direction (to also be abbreviated as "CD") perpendicular to the direction in which they extend. When viewed overhead from the first surface, the ridges 2 extend in the machine direction while the outer edges thereof repeatedly meander. Meandering as described here refers to the actual distance along the ridge outer edges between any arbitrary two points on the outer edges of the ridges at least 15 mm apart in terms of linear distance (referred to as "substantial length") being greater than linear distance in the case of connecting the two points with a straight line (referred to as "apparent length"), and the degree of meandering is defined with the equation indicated below.

```
Degree of meandering = substantial length/apparent
length
```

In addition, in the case of focusing on a single ridge, although two outer edges are present in the ridge on both sides of the center in the cross-machine direction thereof, among the two outer edges, the outer edge having the greater degree of meandering is selected for calculating degree of meandering.

Furthermore, the phase difference between the two peripheral edges may be symmetrical or asymmetrical. The ridge outer edges described here define the contour of a portion equivalent to 50% of the ridge height when the non-woven fabric sheet is viewed overhead.

The methods for specifying ridge outer edges and measuring substantial length and apparent length are as described below.

After having incorporated images within a range of 30 mm x 30 mm using a 3D laser displacement gauge (Keyence Corp.), the images are processed according to the following procedure using dedicated image analysis software.

(1) Tilt correction by image correction (automated).
(2) Smoothing by image correction (15 x 15, selected five times).
(3) Portions equal to 50% or less of ridges blackened to facilitate reading of borderlines by setting 50% of actual range displayed according to upper and lower limit settings (= ridge height) to lower limit.
(4) Borderlines of printouts of images processed in the above manner carefully traced followed by measurement of substantial length and apparent length.
(5) Measurement carried out at 20 locations or more followed by use of average value thereof.

From the viewpoint of increasing surface area, the degree of meandering is preferably 1.1 to 2.0 and more preferably 1.2 to 1.5. In the case of being below these ranges, a roughly linear shape results and there is hardly any effect on increasing surface area in order to the amount of entrapped stool. In addition, if the degree of meandering exceeds the above ranges, it becomes difficult to form ridges and grooves.

From the viewpoints of separation of the skin from highly viscous liquid absorbed by the non-woven fabric sheet 1 or entrapped in the grooves thereof and feel on the skin, the thickness $t_2$ of the ridges (see FIG. 3) is preferably 0.3 mm to 5 mm and more preferably 0.5 mm to 3 mm. If the thickness $t_2$ exceeds these ranges, the awareness of the presence of the absorbent article on the skin ends up increasing. In addition, if the thickness $t_2$ is below these ranges, separation between the highly viscous liquid that has been absorbed or entrapped in the grooves and the skin is inadequate, thereby preventing a reduction in the amount adhered to the skin.

From the viewpoints of increasing the substantial surface area of the non-woven fabric sheet 1 and incorporating high viscous liquid in the grooves, a height difference D between the tops and bottoms of the grooves (see FIG. 3) is preferably 0.1 mm to 3 mm and more preferably 0.3 mm to 2 mm. In the case the height difference D exceeds these ranges, the ridge height inevitably increases and the awareness of the presence of the absorbent article on the skin ends up increasing. In addition, if the height difference D is below these ranges, the amount incorporated in the grooves ends up becoming extremely low.

The ridge thickness $t_2$ and the height difference D between the tops and bottoms of the ridges are measured using a 3D laser displacement gauge (Keyence Corp.).

Although the ridge thickness $t_2$ may be constant over the direction (MD) in which the ridges extend, from the viewpoints of feel on the skin and entrapment of high viscous liquid, sites where the ridge height $t_2$ is relatively high over the machine direction and sites where the ridge height $t_2$ is relatively low are preferably alternately formed. A height difference $D_2$ between relatively high portions and relatively low portions (see FIG. 4) is preferably 0.1 mm to 3 mm and more preferably 0.3 mm to 2 mm. In the case the height difference $D_2$ exceeds these ranges, the ridge height $t_2$ inevitably becomes high and awareness of the presence of the absorbent article on the skin ends up increasing. In addition, in the case the height difference $D_2$ is below these ranges, there is hardly any effect on increasing surface area in order to enhance the amount of entrapped stool.

From the viewpoints of re-adhesion of waste liquid to the skin in the surface sheet 1 and feel on the skin, a width $w_2$ of ridges in the cross-machine direction of the non-woven fabric sheet 1 (see FIG. 3) is preferably within the range of 1 mm to 10 mm and more preferably within the range of 2 mm to 5 mm. In the case the width $w_2$ exceeds these ranges, the region that contacts the skin of a wearer increases, which together with causing a feeling of stickiness or increasing a feeling of irritation attributable to chafing, results in the risk of highly viscous liquid present on the ridges becoming re-adhered to the skin. In addition, in the case the width $w_2$ is below these ranges, it ends up becoming difficult to form the ridges and grooves.

From the viewpoints of re-adhesion of waste liquid to the skin and entrapment of highly viscous liquid, a width $w_3$ of the grooves in the cross-machine direction of the non-woven fabric sheet 1 (see FIG. 3) is preferably such that wide portions and narrow portions are repeated within a range of 0.5 mm to 7 mm and more preferably within a range of 1 mm to 3 mm. In the case the width $w_3$ exceeds these ranges, the skin ends up cutting into the grooves and the region that contacts the skin increases. In addition, in the case the width $w_3$ is below these ranges, entrapment capacity of highly viscous liquid into the recesses ends up becoming extremely low.

Three-dimensional, random orientation is enhanced by at least a portion of the fibers that compose the ridges 2 generating curvature or bending. As a result, the formation gaps between fibers capable of entrapping stool and the maintaining of gaps between fibers when compressed can be enhanced.

Measurement of fiber orientation can be carried out in the manner described below using the VHX-100 Digital Microscope manufactured by Keyence Corp. (1) A sample is placed on an observation stage so that the machine direction is the longitudinal direction, (2) the focus of a lens is aligned with the fibers closest to the front of the sample while excluding those fibers irregularly protruding towards the front, and (3) a 3D image of the sample is generated on a PC screen by setting the depth of field (depth). Next, (4) the 3D image is converted to a 2D image, (5) a plurality of parallel lines are drawn on the screen that suitably equally divide the measuring range in the machine direction. (6) Fiber orientation in each of the divided cells formed by drawing the parallel lines is observed to be in the machine direction or cross-machine direction, and the number of fibers oriented in each direction is measured. Finally, (7) the ratio of the number of fibers oriented in the machine direction and the ratio of the number of fibers oriented in the cross-machine direction to the total number of fibers within the set range are measured and calculated.

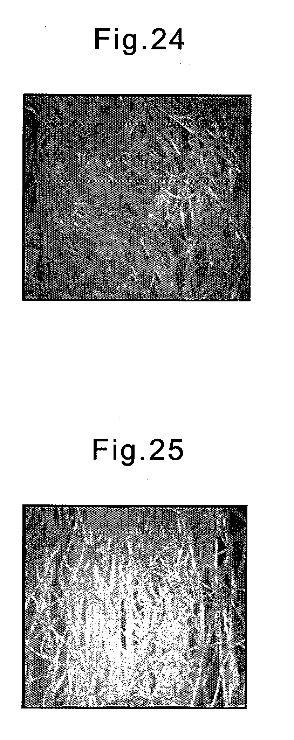

FIG. 24 shows the fiber orientation of the ridges 2 of a non-woven fabric sheet of Example 3, while FIG. 25 shows the fiber orientation of the ridges 2 in a non-woven fabric sheet of Comparative Example 1. The photographs of FIGS. 24 and 25 are photographs taken in step (4) above during measurement of fiber orientation as described above.

As shown in FIGS. 3 and 4, the non-woven fabric sheet 1 has a multi-layer structure having a first fibrous layer 4 located on the side of the first surface 1a and a second fibrous layer 5 located on the side of the second surface 1b. The first fibrous layer 4 and the second fibrous layer 5 are integrated into a single unit by a method such as fiber entanglement or heat fusion at the surface where both are opposed.

The first fibrous layer 4 is composed of a fibrous layer containing heat-fusible fibers as essential fibers, while the second fibrous layer 5 is composed of a fibrous layer containing latent crimpable fibers as essential fibers.

Fibers composed of a thermoplastic resin are preferably used for the fibers that compose the first fibrous layer 4. Examples of thermoplastic resins include polyolefins such as a polyethylene or polypropylene, polyesters such as polyethylene terephthalate, and polyamides. In addition, core-sheath or side-by-side type composite fibers consisting of a combination of these thermoplastic resins can also be used.

From the viewpoint of causing the ridges 2 to meander by generating curvature or bending along the machine direction due to shrinkage of the second fibrous layer 5 to be subsequently described, the heat-fusible fibers that compose the first fibrous layer 4 preferably do not substantially demonstrate heat shrinkage, or have a crimping starting temperature that is higher than the crimping starting temperature of the latent crimpable fibers of the second fibrous layer 5. Moreover, from the same viewpoint, the fibers that compose the first fibrous layer 4 preferably have a temperature at which they are fused and fixed that is higher than the temperature at which the latent crimpable fibers of the second fibrous layer 5 demonstrate crimping.

In addition, from the viewpoints of feel on the skin and absorbency of the non-woven fabric sheet 1, the fineness of the heat-fusible fibers composing the first fibrous layer 4 is preferably 1 dtex to 5 dtex and more preferably 2 dtex to 4 dtex. In addition, from the viewpoint of proper carding, the fiber length is preferably 15 mm to 65 mm and more preferably 38 mm to 51 mm.

Moreover, the first fibrous layer 4 may be composed of one type or two or more types of heat-fusible fibers, or may be composed by containing other fibers that do not heat-fuse with the heat-fusible fibers in addition to the heat-fusible fibers. For example, one or more types of fibers can be arbitrarily selected and used from among regenerated fibers such as rayon fibers, semi-synthetic fibers such as acetate fibers, natural fibers such as cotton or wool fibers, and synthetic fibers such as polypropylene, polyethylene, polyester, polyamide, polyvinyl chloride or vinylon fibers. In addition, there are no limitations on the cross-sectional shape and so forth of the fibers used, and split-type composite fibers or modified cross-section fibers can be used arbitrarily. In this case, the amount of fibers able to heat-fuse the intersections of the fibers is preferably 30% by weight to 95% by weight and more preferably 70% by weight to 90% by weight of the total weight of the first fibrous layer 4.

On the other hand, the latent crimpable fibers that compose the second fibrous layer 5 are composed of, for example, eccentric core-sheath type composite fibers or side-by-side composite fibers composed of two types of thermoplastic resins having different shrinkage rates. In addition, from the viewpoint of causing the ridges 2 to meander by shrinking the second fibrous layer 5 and generating curvature or bending in the fibers that compose the first fibrous layer 4 in the machine direction, latent crimpable fibers are preferably used that have a web shrinkage rate of at least 40%. Examples include composite fibers of polyolefin-polypropylene copolymers and polypropylene. In addition, from the viewpoint of ensuring favorable liquid permeability, the fineness of the fibers used is preferably 1 dtex to 11 dtex and more preferably 2 dtex to 6 dtex. In addition, from the viewpoint of proper carding, the length of the fibers used is preferably 15 mm to 65 mm and more preferably 38 mm to 51 mm.

The second fibrous layer 5 may be composed of one layer or two or more layers of latent crimpable fibers, or may be composed by containing other fibers that do not heat-fuse with the latent crimpable fibers in addition to the latent crimpable fibers. For example, one or more types of fibers can be arbitrarily selected and used from among regenerated fibers such as rayon fibers, semi-synthetic fibers such as acetate fibers, natural fibers such as cotton or wool fibers, and synthetic fibers such as polypropylene, polyethylene, polyester, polyamide, polyvinyl chloride or vinylon fibers. In addition, there are no limitations on the cross-sectional shape and so forth of the fibers used, and split-type composite fibers or modified cross-section fibers can be used arbitrarily. In this case, from the viewpoint of demonstrating adequate heat shrinkage, the latent crimpable fibers are preferably used at 10% by weight to 95% by weight and more preferably at 60% by weight to 80% by weight.

The basis weight following the shrinkage step of the non-woven fabric sheet 1 is preferably 35 g/m$^2$ to 150 g/m$^2$ and more preferably 40 g/m$^2$ to 80 g/m$^2$ from the viewpoint of feel during use when incorporating in a product. For example, basis weight of the first fibrous layer 4 following the shrinkage step can be preferably 20 g/m$^2$ to 100 g/m$^2$ and more preferably 30 g/m$^2$ to 60 g/m$^2$, while basis weight of the second fibrous layer 5 following the shrinkage step can be preferably 15 g/m$^2$ to 70 g/m$^2$ and more preferably 20 g/m$^2$ to 50 g/m$^2$.

In a preferable configuration thereof, the ridges 2 are mainly occupied by the first fibrous layer 4. For example, 50% to 80% of the ridges 2 is composed of the first fibrous layer 4 based on area in the cross-sectional shape shown in FIG. 3, while the remainder is composed of the second fibrous layer 5. On the other hand, the difference in ratios at which the first fibrous layer 4 and the second fibrous layer 5 occupy the grooves 3 based on area in the cross-sectional shape shown in FIG. 3 is not as large as that of the ridges 2. For example, 40% to 70% of the grooves 3 is composed of the first fibrous layer based on area, while the remainder is composed of the second fibrous layer 5. The reason for the ratio of the first fibrous layer 4 being larger in the ridges 2 is because this is effective for causing meandering of the ridges 2 and further increasing surface area as a result of generating curvature or bending of the fibers that compose the first fibrous layer 4 along the machine direction.

The non-woven fabric sheet 1 is preferably hydrophilic. The non-woven fabric sheet 1 can be made to be hydrophilic by, for example, using fibers that have been treated with a hydrophilizing agent for the raw material thereof. In addition, a method can also be used in which fibers incorporating a hydrophilizing agent are used for the raw material. Moreover, a method can also be used that uses fibers inherently possessing hydrophilicity, such as natural fibers or semi-natural fibers. The non-woven fabric sheet 1 can also be made to be hydrophilic following the production thereof by coating with a surfactant.

Next, an explanation is provided of a method for producing the non-woven fabric sheet of the present invention.

The production method of the present invention comprises the following steps; however, steps e and h are not required:

a) a step for opening a fiber assembly containing latent crimpable fibers by passing through a carding machine to form a web containing latent crimpable fibers,

b) a step for opening a fiber assembly containing heat-fusible fibers that do not demonstrate crimping at the crimping starting temperature of the latent crimpable fibers by passing through a carding machine to form a web containing heat-fusible fibers,

c) a step for superimposing the web containing latent crimpable fibers and the web containing heat-fusible fibers to form a laminated web,

d) a step for realigning the fibers so that ridges and grooves are formed in the laminated web, and portions in which the degree of fiber entanglement in the direction in which the ridges are continuous is relatively high and portions in which it is relatively low are alternately formed,

e) a step for transporting the laminated web obtained in step d to step f,

f) a step for heating the web, in which fibers have been realigned, to cause the latent crimpable fibers to demonstrate crimping,

g) a step for heating the web in which crimping has been demonstrated by the latent crimpable fibers to fuse the heat-fusible fibers at sites where they mutually intersect, and

h) a step for cooling the web obtained in step g.

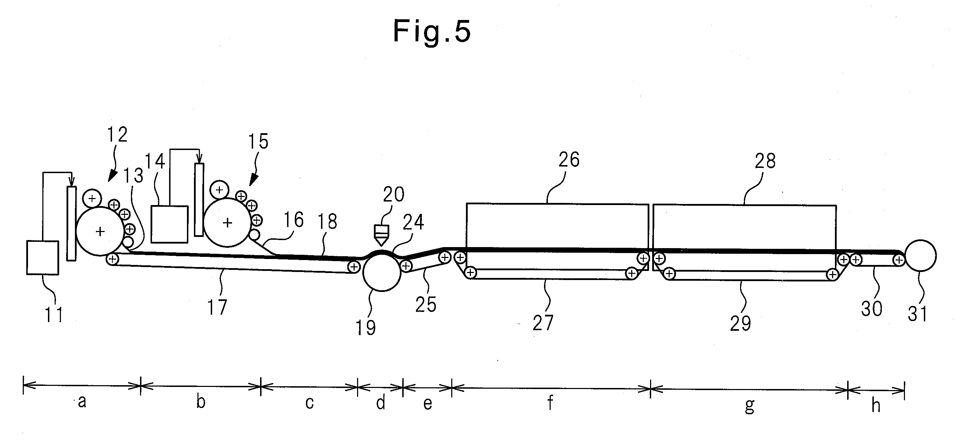

FIG. 5 shows an example of the production steps of the non-woven fabric sheet of the present invention. However, the present invention is not limited to this example.

In FIG. 5, a indicates step a, b indicates step b, c indicates step c, d indicates step d, e indicates step e, f indicates step f, g indicates step g, and h indicates step h.

In step a, a fiber assembly containing latent crimpable fibers is transported from a container 11 to a carding machine 12, and the assembly is opened by passing through the carding machine 12 to form a web 13 containing latent crimpable fibers. The formed web 13 containing latent crimpable fibers is transported by placing on an endless belt 17.

In step b, a fiber assembly containing heat-fusible fibers is transported from a container 14 to a carding machine 15 and opened by passing through the carding machine 15 to form a web 16 containing heat-fusible fibers. The step a and the step b can be carried out in any order.

In step c, the web 13 containing latent crimpable fibers and the web 16 containing heat-fusible fibers are superimposed to form a laminated web 18. Although an aspect is shown in FIG. 5 in which the formed web 16 containing heat-fusible fibers is superimposed on the web 13 containing latent crimpable fibers on the endless belt 17, the web 16 containing heat-fusible fibers is not necessarily required to be superimposed on the web 13 containing latent crimpable fibers, but rather the laminated web 18 may be formed by superimposing the web 13 containing latent crimpable fibers on the web 16 containing heat-fusible fibers. Namely, step a and step b may be carried out in any order, and when transporting the laminated web, the web 16 containing heat-fusible fibers may be on top or the web 13 containing latent crimpable fibers may be on top.

Step d is a step for realigning the fibers so that ridges and grooves are formed in the laminated web, and portions in which the degree of fiber entanglement in the direction in which the ridges are continuous is relatively high and portions in which it is relatively low are alternately formed. Step d is preferably a step in which the laminated web is transported by placing on a support, in which liquid passage portions and liquid blocking portions extending in parallel in the cross-machine direction alternately repeat in the machine direction, while spraying a liquid from a plurality of nozzles arranged in a row in the cross-machine direction onto the side of the laminated web containing heat-fusible fibers to form a plurality of ridges and grooves extending in parallel in the machine direction. Here, the machine direction refers to the direction in which the web is transported in the production process, while the cross-machine direction refers to the direction perpendicular to the machine direction on the web surface. In the following descriptions, the machine direction may be abbreviated as MD while the cross-machine direction may be abbreviated as CD. In addition, the lengthwise direction of the non-woven fabric sheet coincides with the machine direction, while the widthwise direction of the non-woven fabric sheet coincides with the cross-machine direction.

Step d shown in FIG. 5 includes a suction drum 19 that rotates in the machine direction, and a plurality of nozzles 20 arranged in a row in the cross-machine direction. The plurality of nozzles 20 arranged in a row in the cross-machine direction are able to spray a liquid towards the circumferential surface of the suction drum 19, and are separated from the circumferential surface of the suction drum 19 by a required dimension. The plurality of nozzles 20 arranged in a row in the cross-machine direction are attached at prescribed intervals to a pipe (not shown) extending in the axial direction of the suction drum 19, namely the cross-machine direction CD.

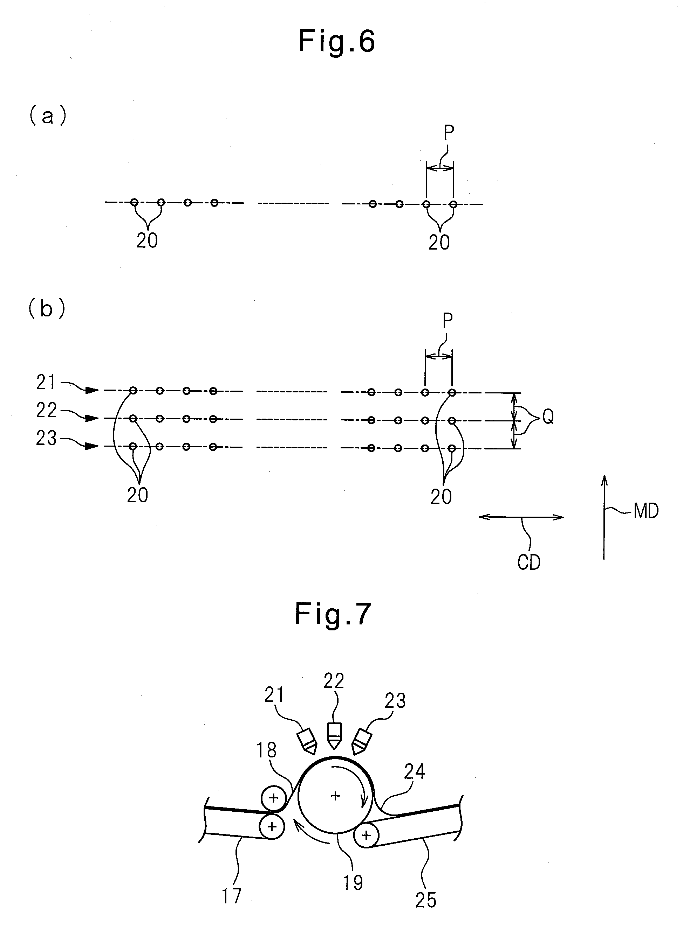

Although the plurality of nozzles 20 arranged in a row in the cross-machine direction may consist of a single row as shown in FIG. 6(a), the nozzles are preferably composed by arranging in two or more rows from the viewpoint of fiber penetrability. For example, the nozzles may be composed of nozzle rows 21, 22 and 23 as shown in FIG. 6(b), and in a preferable example of an attached state thereof, the nozzles 20 are adjusted so as to be located along the same line in the machine direction MD in each of the nozzle rows 21, 22 and 23. In addition, the nozzle rows 21, 22 and 23 can be arranged while separated by intervals of 30° each in the circumferential direction of the suction drum 19 as shown in FIG. 7, and the nozzles 20 of each of the nozzle rows 21, 22 and 23 can be attached to a pipe at a pitch P of 5 mm in the cross-machine direction, for example. Liquid of a prescribed temperature can be sprayed at a prescribed spray volume from the nozzle rows 21, 22 and 23. The liquid sprayed from the plurality of nozzles 20 is adjusted so as not to disturb the distribution state of the fibers in the web as a result of mutual interference between the liquid per se or liquid from the nozzles 20. In order to accomplish this, in the case, for example, the web having a total basis weight of 35 g/m$^2$ passes over the circumferential surface of the suction drum 19 having a diameter of 500 mm in 0.5 seconds, the nozzles 20 of each of the nozzle rows 21, 22 and 23 are arranged at a pitch P of 5 mm in the cross-machine direction, and the distance from the circumferential surface of the suction drum 19 is adjusted to 5 mm to 8 mm, the web 18 preferably passes beneath the nozzles 20 arranged in a row in the cross-machine direction after adjusting the thickness to about 2 mm to 5 mm with the suction of the suction drum 19. The aperture of the nozzles 20 used at this time is preferably about 0.5 mm to 1.5 mm, the spraying rate of liquid from the nozzles 20 is preferably 50 m/sec to 700 m/sec, and the suctioning force of the suction drum 19 is preferably an air blowing rate of 2 m/sec to 7 m/sec.

Examples of the liquid sprayed from the nozzles include a gas adjusted to normal temperature or a prescribed temperature, and an aerosol in which fine particles of a gas or liquid are contained in the gas. Examples of gases include air and nitrogen. In addition, the gas may contain a vapor of a liquid such as water vapor. An aerosol refers to a dispersion of a liquid or solid in a gas, and the following lists examples thereof. Namely, examples of aerosols include inks for coloring, softeners such as silicone for enhancing flexibility, hydrophilic or water-repellent activators for controlling antistatic properties and wettability, inorganic fillers such as titanium dioxide or barium sulfate for enhancing energy of the liquid, powder bonds such as polyethylene for enhancing liquid energy as well as enhancing the formation and maintenance of surface irregularities during heat treatment, antihistamine agents such as diphenhydramine hydrochloride or isopropyl methylphenol for preventing itching, moisturizers and dispersions containing disinfectants. Here, solids include gelled solids.

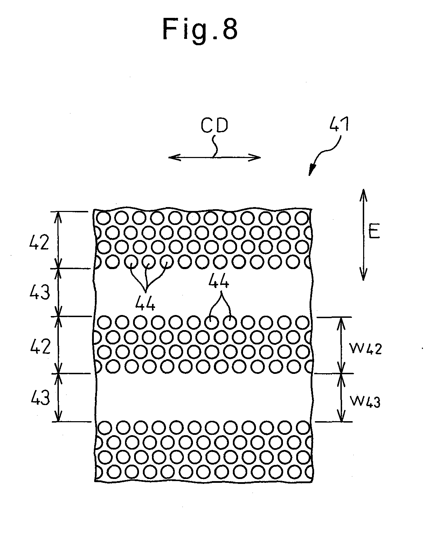

A molding plate is attached to the circumferential surface of the suction drum 19. FIG. 8 shows an example of a molding plate. A molding plate 41 has liquid passage portions 42 and liquid blocking portions 43 alternately formed in the circumferential direction E (machine direction) of the suction drum 19, a plurality of holes 44 are formed in the liquid passage portions 42, and the holes 44 are connected to a suction mechanism (not shown) of the suction drum 19.

In one example of the molding plate 41, a dimension $w_{42}$ in the circumferential direction E of the liquid passage portions 42 of the molding plate is 2 mm to 3 mm, the liquid passage portions 42 extend over nearly the entire axial of the suction drum 19, namely the cross-machine direction, and a large number of the holes 44 having a diameter of 0.2 mm to 1 mm are formed so as to form an aperture ratio of 15% to 30% with respect to the surface area of the liquid passage portions 42 of the molding plate. The liquid blocking portions 43 of the molding plate have a dimension $w_{43}$ in

the circumferential direction E of 1.5 mm to 3 mm, and extend over the entire axial direction of the suction drum 19. The peripheral velocity of the suction drum 19 to which the molding plate is attached is the same as the transport speed of the web.

The web is then placed on the circumferential surface of the suction drum 19 and passes below the nozzles 20. Although a liquid is sprayed towards the web from the nozzles 20, in the suction drum 19, the suction acts to suction the liquid. In the web that has been sprayed with liquid, fibers directly below the nozzles 20 move in parallel in the cross-machine direction and accumulate between the adjacent nozzles 20 to form the ridges 2. On the other hand, the grooves 3 are formed directly below the nozzles 20.

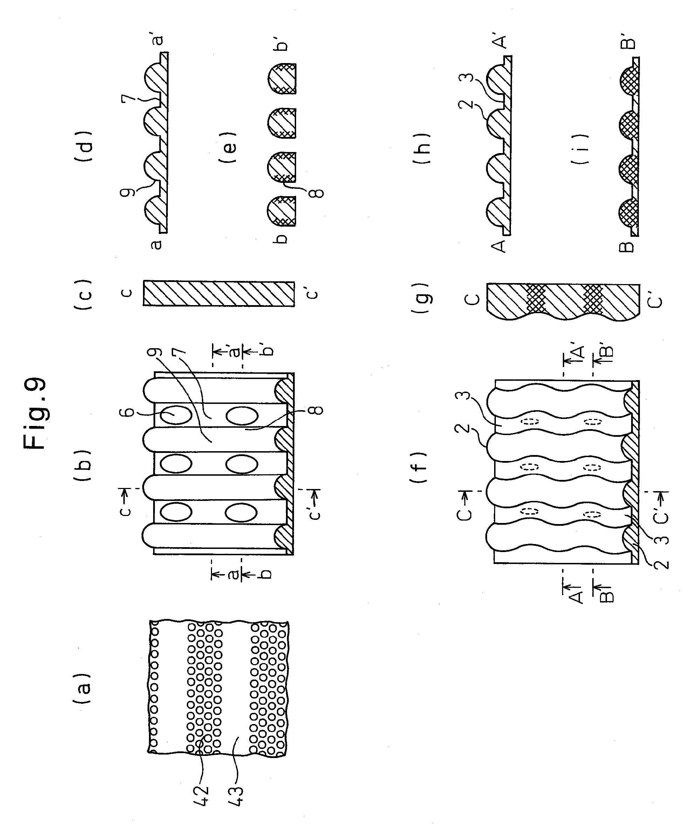

FIG. 9 is a drawing for explaining the process by which the ridges 2 and the grooves 3 of the non-woven fabric sheet of the present invention are formed. FIG. 9(a) shows a molding plate, FIG. 9(b) is an overhead perspective view of a non-woven fabric sheet prior to heat treatment obtained in step (d), FIG. 9(c) is a cross-sectional view taken along line c-c' of FIG. 9(b),

FIG. 9(d) is a cross-sectional view taken along line a-a' of FIG. 9(b), and FIG. 9(e) is a cross-sectional view taken along line b-b' of FIG. 9(b). FIG. 9(f) is an overhead perspective view of a non-woven fabric sheet following heat treatment obtained in step (f), FIG. 9(g) is a cross-sectional view taken along line C-C' of FIG. 9(f), FIG. 9(h) is a cross-sectional view taken along line A-A' of FIG. 9(f), and FIG. 9(i) is a cross-sectional view taken along line B-B' of FIG. 9(f).

In the liquid passage portions 43 of the molding plate 41 forming the circumferential surface of the suction drum 19, liquid that has been sprayed towards the web does not proceed to the inside of the suction drum 19, but rather flows in the cross-machine direction along the surface of the molding plate 41. Due to this liquid, when fibers placed on the liquid blocking portions 43 are moved in the cross-machine direction, thin-walled portions 6 corresponding to the holes 44 are formed in the web, and when nearly all of the fibers placed on the liquid blocking portions 43 have moved in the cross-machine direction, through holes 6 corresponding to the holes 44 are formed in the web. In addition, when the majority of the liquid sprayed towards the fibers placed on the liquid passage portions 42 of the molding plate 41 has proceed through the holes 44 of the molding plate 41 to the inside of the suction drum 19, a portion of the fibers remain below the nozzles 20 without moving in the cross-machine direction, and bridges 7 that connect the adjacent ridges 2 are formed.

The amount by which the fibers are pushed aside at the portions corresponding to the fluid blocking portions 43 of the molding plate is relatively larger than the fluid passage portions 42 of the molding plate, and in the ridges 2 formed as a result thereof, fiber assembly density at those portions corresponding to the fluid blocking portions 43 of the molding plate are relatively higher than those portions corresponding to the fluid passage portions 42 of the molding plate, and the degree of entanglement also increases. Portions 8 where this fiber assembly density is relatively high and portions 9 where it is relatively low are alternately arranged in the machine direction due to the pattern of the molding plate.

Step e is a step for transporting a web 24 obtained in step d to step f. In step e, the web 24 is transported by being placed on an endless belt 25. The transport speed in step e is either the same as the transport speed in step d or slightly faster than the transport speed in step d. Step e is not necessarily required, and the web 24 in which fibers have been realigned in step d may also be sent directly from step d to step f. However, step e is preferably provided in order to stably transport the web 24.

Step f is a first heat treatment step for expressing crimping of the latent crimpable fibers of the second fibrous layer.

The web obtained in step d is sent to the first heat treatment step f after going through the transport step e as necessary. In the first heat treatment step f, a first heat treatment dryer 26 is provided, the web 24 is placed on an endless belt 27, and the web 24 is passed through the first heat treatment dryer 26 to carry out heat treatment therein.

In the first heat treatment step f, fibers of the second fibrous layer are made to express crimping by carrying out heat treatment under heating conditions within a range at which shrinkage of the fibers of the second fibrous layer begins and fibers of the second fibrous layer are not fused and fixed. Here, since the first fibrous layer does not shrink when the second fibrous layer shrinks in the machine direction, curvature or bending occurs in the machine direction in the fibers that compose the first fibrous layer. At this time, in the case the ridges are composed of portions where fiber assembly density is relatively high and portions where it is relatively low as previously described, the fibers easily curve or bend in the machine direction at those portions where fiber assembly density is relatively low and the degree of entanglement is low, and as a result thereof, meandering portions and wide portions are formed in the ridges. On the other hand, at those portions where fiber assembly density is relatively high and the degree of entanglement is high, since the degree of freedom of the fibers is low, it is difficult for curvature or bending to occur in the machine direction, and as a result thereof, meandering starting points and narrow portions are formed in the ridges.

Moreover, curvature or bending of fibers at those portions of the ridges where fiber assembly density is relatively low and the degree of entanglement is low not only increase in width in the cross-machine direction, but also impart bulkiness to the ridges, thereby resulting in the formation of relatively high portions in the ridges. In addition, curvature or bending of fibers at those portions of the ridges where fiber assembly density is relatively high and the degree of entanglement is high occurs randomly according to the fiber orientation at those portions.

In addition, in the case tension has acted in the machine direction on the web within the first heat treatment dryer 26, since shrinkage of the second fibrous layer resulting from expression of crimping by the latent crimpable fibers therein occurs easily in the cross-machine direction CD where the degree of freedom is relatively high, adequate shrinkage in the machine direction for causing curvature or bending of the fibers of the first fibrous layer in the machine direction cannot be obtained. Therefore, from the viewpoint of actively causing the second fibrous layer to shrink in the machine direction, heat treatment is carried out in a state in which resistance to a force acting to cause shrinkage in the machine direction due to crimping by the latent crimpable fibers is small. Examples of specific methods include a method in which the transport speed of step f is made to be slower than the transport speed of the previous step, and a method that uses a floating dryer.

The previous step in the method in which the transport speed of step f is made to be slower than the transport speed of the previous step refers to step e when step e is provided or step d when step e is not provided. As a result of making the transport speed of the first heat treatment step f to be slower than the transport speed of the previous step, resistance to a force acting to cause shrinkage in the machine direction due to crimping by the latent crimpable fibers can be made to be small, adequate shrinkage in the machine direction is obtained resulting from expression of crimping by the latent crimpable fibers of the second fibrous layer, and meandering ridges are formed easily. For example, the transport speed in the first heat treatment step f is preferably 80% to 95% of the transport speed of the previous step for the range over which adequate shrinkage force is demonstrated in the machine direction and the sheet does not become bent or folded.

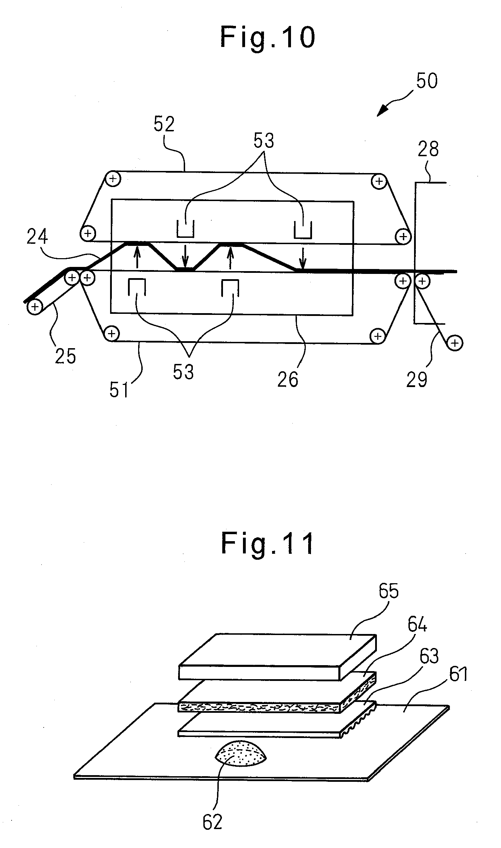

Although one component of resistance to shrinkage resulting from expression of crimping by the latent crimpable fibers is line tension during transport, another component is friction with the transport conveyor. In order to reduce this friction, a floating dryer 50 can be used as shown in FIG. 10. The floating dryer 50 has mesh conveyors 51 and 52 installed above and below while mutually separated, a plurality of hot air outlets 53 are provided to the inside of the mesh conveyors 51 and 52 (on the opposite side from the web 24), and heat treatment is carried out while blowing hot air towards one mesh conveyor 52 or 51 and moving the web 24 to the other mesh conveyor 52 or 51. As shown in FIG. 10, after having blown hot air from the lower mesh conveyor 51, hot air is then blown from the upper mesh conveyor 52, and as a result of alternatively blowing hot air from above and below, the web 24 is moved up and down, portions are formed that do not contact the mesh conveyors 51 and 52, and resistance to the force that attempts to cause shrinkage in the machine direction due to expression of crimping by the latent crimpable fibers can be reduced, thereby making this method preferable for shrinking in both the machine direction and cross-machine direction. The floating dryer can be used in combination with a method for making the transport speed of the first heat treatment step f slower than the transport speed of the previous step, and is preferably used in combination therewith.

The temperature of the first heat treatment step f is within a range that does not exceed the fusing temperature of the heat-fusible fibers of the first fibrous layer, is preferably a temperature 0°C to +50°C higher, and more preferably a temperature +10°C to +40°C higher, than the temperature at which the latent crimpable fibers of the second fibrous layer express crimping. For example, in the case the temperature at which the latent crimpable fibers of the second fibrous layer express crimping is 80°C and the melting point of the heat-fusible fibers of the first fibrous layer is 130°C, then the temperature of the first heat treatment dryer 26 is preferably 80°C to 130°C and more preferably 90°C to 120°C.

Step g is a second heat treatment step for fusing the heat-fusible fibers of the first fibrous layer at those sites where the fibers mutually intersect. In the second heat treatment step g, a second heat treatment dryer 28 is provided, and the web in which the latent crimpable fibers have been crimped in step f is placed on an endless belt 29, and the belt is passed through the second heat treatment dryer 28 where it undergoes heat treatment therein. In the second heat treatment step g, heat treatment is carried out at a temperature equal to or higher than the fusing temperature of the heat-fusible fibers of the first fibrous layer, and as a result of the heat-fusible fibers fusing at those sites where the fibers mutually intersect, the ridges of the first fibrous layer formed in steps d to f are fixed.

The temperature of the second heat treatment step g is preferably -10°C to +40°C higher and more preferably 0°C to +20°C higher than the melting point of the heat-fusible fibers of the first fibrous layer. For example, in the case the melting point of the heat-fusible fibers of the first fibrous layer is 130°C, then the temperature of the second heat treatment dryer 28 is preferably 120°C to 170°C and more preferably 130°C to 150°C.

Step h is a step for cooling the web in which the heat-fusible fibers have been fused in step g. After having passed through the second heat treatment dryer 28 of step g, the web is transported by being placed on an endless belt 30 while allowing to cool at room temperature. As a result of this cooling, fusion of the heat-fusible fibers of the first fibrous layer is fixed at those sites where the fibers mutually intersect and the first fibrous layer is fixed. The cooled web is then wound onto a roller 31.

The non-woven fabric sheet of the present invention can be preferably used as a member of absorbent articles such as disposable diapers, sanitary napkins or cleansing wipes. In particular, the non-woven fabric sheet of the present invention can be preferably used in the surface sheet, flaps or back sheet and the like of disposable diapers. In addition, the non-woven fabric sheet of the present invention can also be preferably used to produce cleansing wipes. In the case of using the non-woven fabric sheet of the present invention as a surface sheet of absorbent articles such as disposable diapers or sanitary napkins, the first surface of the non-woven fabric sheet (surface in which ridges and grooves are formed) is used as the surface that contacts the skin when worn. In the case of using the non-woven fabric sheet of the present invention in a portion of a surface sheet of a disposable diaper, the non-woven fabric sheet is preferably at least arranged farther towards front from the center in the machine direction.

When a non-woven fabric sheet of the present invention is viewed overhead from the first surface, substantial surface area per unit projected area increases as a result of the ridge outer edges repeatedly meandering while extending in the machine direction, thereby making it possible to increase the opportunities for contact with the target highly viscous waste matter.

In addition, in the case in which sites where ridge height is relatively high over the machine direction and sites where ridge height is relatively low are alternately formed, the substantial surface area per unit projected area further increases, thereby further increasing the opportunities for contact with the target highly viscous waste matter.

In addition, since the ridges and grooves are continuous, sheet rigidity in a fixed direction is enhanced and the formed entrapping spaces are resistant to crushing.

In addition, in the case three-dimensional, random orientation is enhanced in the ridges as a result of a portion of the fibers generating curvature or bending, the formation of gaps between fibers capable of entrapping stool and the maintaining of gaps between fibers when compressed are improved.

Although soft stool adhered to the skin is wiped off using a baby wipe or tissue when changing the diapers of newborns, from the viewpoints of reducing the bother of wiping off and cost advantages resulting from reducing the number of wipes or tissues used, there are many cases in which, when having opened up the diaper during changing, after preliminarily wiping using an unsoiled portion of the surface sheet of the diaper (such as the front) or a flap or back sheet, the skin is then cleanly wiped with a baby wipe or tissue. In such cases, the function of the surface sheet, flap or back sheet is required to have the conventional functions of absorption or covering by demonstrating the function of an outer member, but also a function that allows stool adhered to the skin to be wiped off. As a result of having the functions described above, the non-woven fabric sheet of the present invention enables highly viscous waste matter to be easily wiped from the skin when changing diapers, thereby reducing the bother of changing diapers. Examples

Example 1

A non-woven fabric sheet was produced using the production device shown in FIG. 5.

In step a, polypropylene/polyolefin-propylene copolymer latent crimpable, side-by-side composite fibers (2.6 dtex, fiber length: 51 mm, weight ratio = 50/50, area shrinkage rate: 60%) were used as fibers for the second fibrous layer, and a web containing the latent crimpable fibers was fabricated having a basis weight of 15 g/m$^2$.

In step b, polyester/polyethylene core-sheath type composite fibers (heat-fusible fibers, 3.0 dtex, fiber length: 51 mm, core:sheath weight ratio = 50/50) were used as fibers for the first fibrous layer, and a web containing the heat-fusible fibers was fabricated having a basis weight of 20 g/m$^2$.

In step c, the web containing the heat-fusible fibers was laminated onto the web containing the latent crimpable fibers.

In step d, the web was sprayed at a flow rate of 250 m/sec using the molding plate shown in FIG. 8 (dimension $w_{42}$ in the circumferential direction of the liquid passage portions = 2.7 mm, diameter of the holes 44 of the liquid passage portions = 0.8 mm (staggered at 45°), aperture ratio of the liquid passage portions = 22%, dimension $d_{43}$ in the circumferential direction of the liquid blocking portions = 2.3 mm), using one row of nozzles having an aperture of 1.0 mm and pitch of 4 mm, and using air at a temperature of about 125°C for the sprayed liquid. The distance from the nozzles to the suction drum was made to be 5.0 mm, and the suction force of the suction drum was made to have a hot air blowing rate of 5 m/s.

In step e, the transport speed was made to be 10 m/min.

In step f, the temperature of the first heat treatment dryer 26 was made to be about 120°C, the hot air blowing rate was made to be 1.0 m/s, and retention time was made to be 10 seconds, and the transport speed was made to be 9 m/min (transport speed equal to 90% of that of step e).

In step g, the temperature of the second heat treatment dryer 28 was made to be 138°C, the hot air blowing rate was made to be 2.5 m/s, the retention time was made to be 10 seconds, and the transport speed was made to be the same as that of step f.

In step h, the web was cooled by allowing to stand at room temperature.

Example 2

A non-woven fabric sheet was produced in the same manner as Example 1 with the exception of making the transport speed in step f to be 8 m/min (transport speed equal to 80% of that of step e).

Example 3

A non-woven fabric sheet was produced in the same manner as Example 1 with the exception of changing the flow rate of the liquid sprayed in step d to 150 m/s and changing the transport speed of step f to 7 m/min (transport speed equal to 70% of that of step e).

Example 4

A non-woven fabric sheet was produced in the same manner as Example 1 with the exception of changing the transport speed of step f to 7 m/min (transport speed equal to 70% of that of step e).

Comparative Example 1

A non-woven fabric sheet was produced in the same manner as Example 1 with the exception of omitting step a, fabricating a web containing heat-fusible fibers having a basis weight of 35 g/m$^2$ in step b, omitting step f, and changing the transport speed of step g to 10 m/min (transport speed equal to 100% of that of step f).

Comparative Example 2

A non-woven fabric sheet was produced in the same manner as Example 1 with the exception of omitting step a, fabricating a web containing heat-fusible fibers having a basis weight of 35 g/m$^2$, omitting step d and step f, and changing the transport speed of step g to 10 m/min (transport speed equal to 100% that of step e).

The non-woven fabric sheets obtained in the examples and comparative examples were evaluated for final fiber density (basis weight), degree of meandering, surface area increase rate, amount of entrapped stool during static loading, amount of entrapped stool during loaded kinetic friction, and stool diffusion area. The results are shown in Table 1. Furthermore, the methods used to evaluate each of the parameters are described below.

[Final Fiber Density (Basis Weight)]

The non-woven fabric sheet was cut to a size measuring 100 mm x 100 mm, and the piece of non-woven fabric sheet was weighed with an electronic balance followed by calculation of weight per square meter. The average of N = 10 measurements was determined.

[Degree of Meandering]

Linear distance in the case of connecting two arbitrary points on the outer edges of ridges separated by 15 mm or more in terms of linear distance with a straight line (to be referred to as "apparent length") and measured distance taken along ridge outer edges between the same two points (to be referred to as "substantial length") were measured followed by calculation of the degree of meandering according to the equation below.

$$\text{Degree of meandering} = \text{substantial length/apparent length}$$

[Surface Area Increase Rate]

After having incorporated images within a range of 30 mm x 30 mm using a 3D laser displacement gauge (Keyence Corp.), surface area was determined according to the procedure indicated below using dedicated image analysis software.

(1) Tilt correction by image correction (automated).
(2) Smoothing by image correction (15 x 15, selected five times).
(3) Measurement of surface area and volume.
(4) "Upper and lower surfaces not included" selected for upper and lower limit settings to eliminate back surface side from measured values.
(5) Entire image selected and measured.
(6) Result of dividing surface area contained in resulting data by selection range used as surface area increase rate.

[Amount of Entrapped Stool during Static Loading]

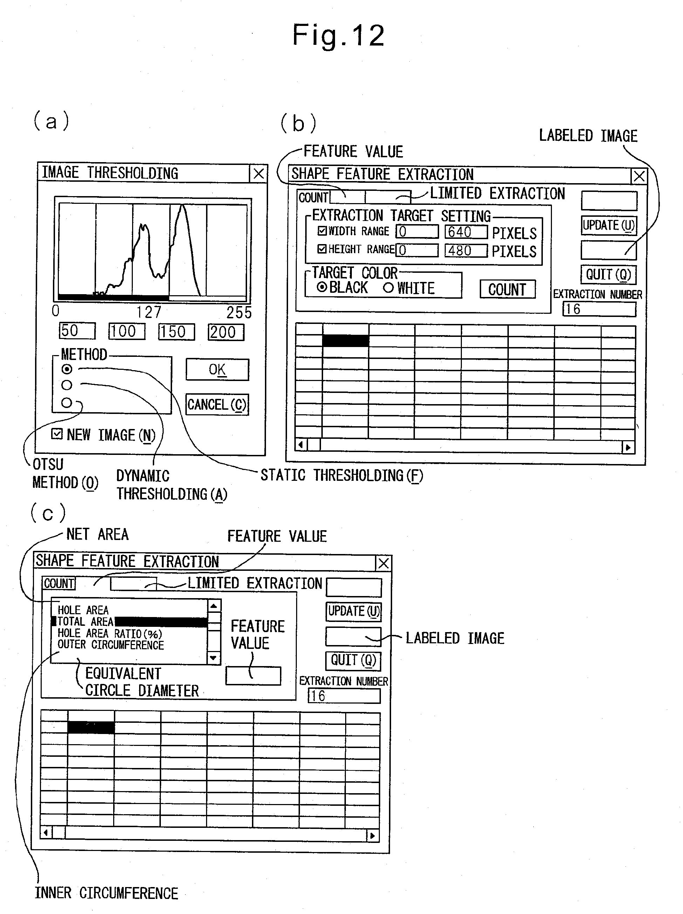

(1) 5.0 g of artificial soft stool 62 were allowed to stand undisturbed on artificial skin 61 placed on a flat surface (see FIG. 11).
Furthermore, artificial soft stool obtained by stirring 112 g of bentonite (Ben-Gel, Hojun Ltd.), 222 g of powdered cellulose (KC Block, Grade W-200, Nippon Paper Chemicals Co., Ltd.), 1666 g of ion exchange water and 1 g of dye (red dye no. 102) with a hand mixer to obtain a homogeneous state free of lumps was used for the artificial soft stool. The artificial soft stool was used after stirring as necessary at the time of use.
(2) The skin contacting side of a sample 63 of a non-woven fabric sheet cut to a size measuring 100 mm x 100 mm was then placed on top of the artificial skin 61, a filter paper 64 (Advantec Inc., size: 100 mm x 100 mm, type: Ananashi, quantity: 1000) was further placed thereon, and a weight 65 weighing 3.5 kg and having a bottom surface measuring 100 mm on a side was further placed thereon, followed by allowing to stand undisturbed for 10 seconds.
(3) The weight 65 was then removed, and the amount of artificial soft stool remaining on the artificial skin 61, the amount of artificial soft stool entrapped by the non-woven fabric sheet sample, and the amount of artificial soft stool that penetrated to the back of the non-woven fabric sheet sample were respectively measured.
(4) The ratios of each of the measured values to the initial amount of 5.0 g were calculated to determine stool adhesion rate, stool entrapment rate and stool penetration rate.
(5) Moreover, following completion of this series of measurements, the non-woven fabric sheet sample was scanned with a scanner, and the resulting image was processed according to the procedure indicated in FIG. 12 with USB Digital Scale image analysis software (Scalar Corp.) to determine diffusion area.

[Amount of Entrapped Stool during Loaded Kinetic Friction]

(1) 5.0 g of artificial soft stool 62 were allowed to stand undisturbed on artificial skin 61 placed on a flat surface (see FIG. 11). The artificial soft stool used was the same as that previously described.
(2) The skin contacting side of a sample 63 of a non-woven fabric sheet cut to a size measuring 100 mm x 100 mm was then placed on top of the artificial skin 61, a filter paper 64 (Advantec Inc., size: 100 mm x 100 mm, type: Ananashi, quantity: 1000) was further placed thereon, and a weight 65 weighing 1 kg and having a bottom surface measuring 100 mm on a side was further placed thereon, followed by moving the non-woven fabric sheet sample 63 at a speed of about 1 cm/s in parallel for 100 mm on the artificial skin 61 with the weight 65 still placed thereon.
(3) The weight 65 was then removed, and the amount of artificial soft stool remaining on the artificial skin 61, the amount of artificial soft stool entrapped by the non-woven fabric sheet sample, and the amount of artificial soft stool that penetrated to the back of the non-woven fabric sheet sample were respectively measured.
(4) The ratios of each of the measured values to the initial amount of 5.0 g were calculated to determine stool adhesion rate, stool entrapment rate and stool penetration rate.
(5) Moreover, following completion of this series of measurements, the non-woven fabric sheet sample was scanned with a scanner, and the resulting image was processed in the same manner as previously described with USB Digital Scale image analysis software (Scalar Corp.) to determine diffusion area.

Table 1

| | Ex.1 | Ex.2 | Ex.3 | Ex.4 | Comp. Ex.1 | Comp. Ex.2 |
|---|---|---|---|---|---|---|
| Final density (g/m$^2$) | 42.7 | 47.6 | 56.0 | 49.8 | 34.8 | 34.7 |
| Degree of meandering | 1.1 | 1.2 | 1.2 | 1.2 | 1.0 | -- |
| Surface area increase rate | 1.5 | 1.6 | 1.7 | 1.7 | 1.3 | 1.0 |
| Amount of entrapped stool during static loading | | | | | | |
| Stool adhesion rate (%) | 9 | 7 | 5 | 6 | 26 | 60 |
| Stool entrapment rate (%) | 72 | 74 | 80 | 79 | 44 | 11 |
| Stool penetration rate (%) | 19 | 20 | 15 | 14 | 30 | 30 |

(continued)

|  | Ex.1 | Ex.2 | Ex.3 | Ex.4 | Comp. Ex.1 | Comp. Ex.2 |
|---|---|---|---|---|---|---|
| Diffusion area ($cm^2$) | 28.3 | 25.9 | 22.6 | 22.9 | 29.9 | 50.8 |
| Amount of entrapped stool during loaded kinetic friction | | | | | | |
| Stool adhesion rate (%) | 16 | 14 | 10 | 12 | 16 | 17 |
| Stool entrapment rate (%) | 77 | 80 | 88 | 84 | 73 | 72 |
| Stool penetration rate (%) | 7 | 6 | 3 | 5 | 10 | 11 |
| Diffusion area ($cm^2$) | 28.3 | 26.2 | 22.9 | 24.8 | 38.2 | 51.0 |

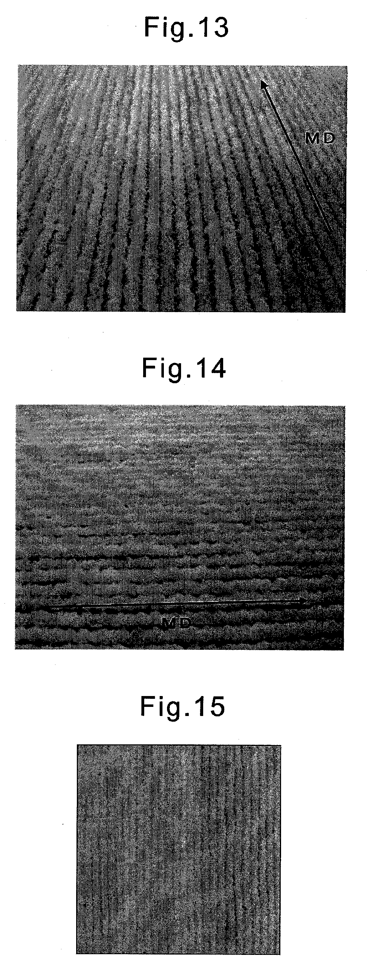

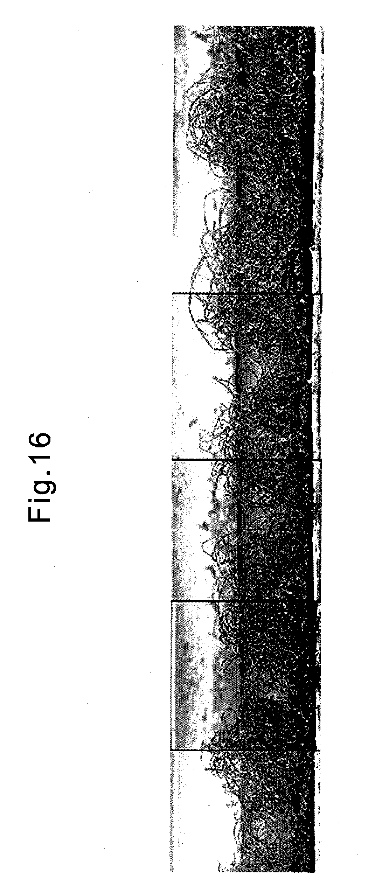

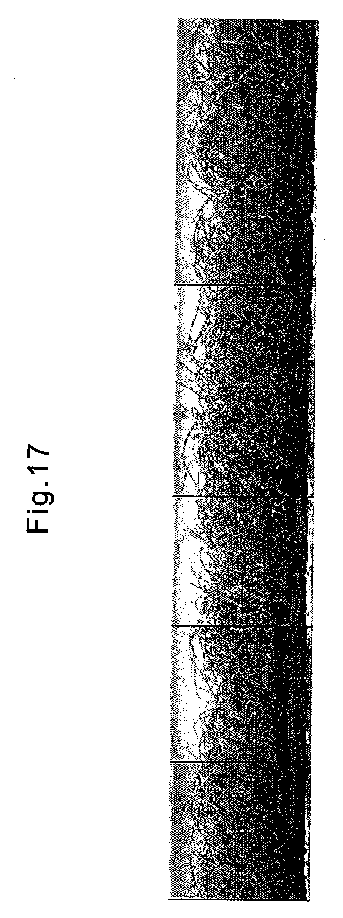

An overhead perspective photograph (digital camera) of the first surface side of the non-woven fabric sheet of Example 3 as viewed from the machine direction is shown in FIG. 13, an overhead perspective photograph (digital camera) of the first surface side of the non-woven fabric sheet of Example 3 as viewed from the cross-machine direction is shown in FIG. 14, an overhead photograph (digital camera, scanner incorporation, actual size) of a first surface is shown in FIG. 15, a photomicrograph of a cross-section in the cross-machine direction (magnification: 20X) is shown in FIG. 16, and a photomicrograph of a cross-section parallel to the machine direction that passes through the tops of the ridges (magnification: 20X) is shown in FIG. 17.

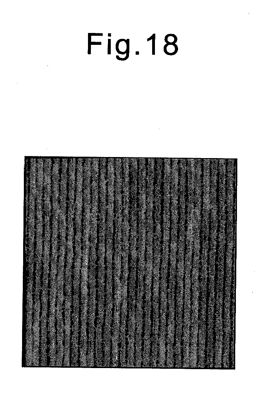

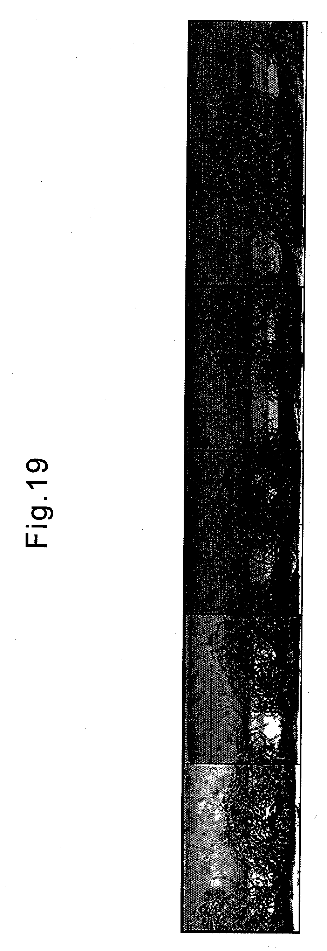

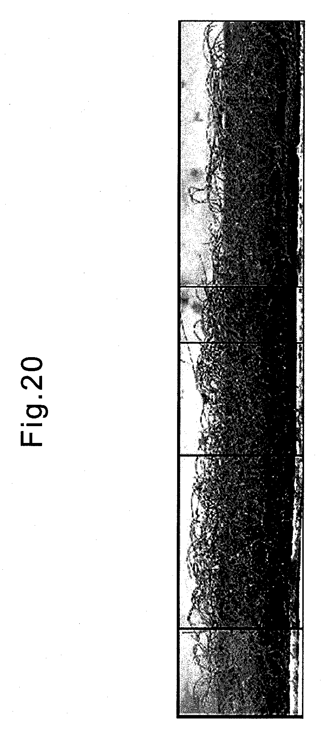

An overhead photograph of the first surface of the non-woven fabric sheet of Example 1 (digital camera, scanner incorporation, actual size) is shown in FIG. 18, a photomicrograph of a cross-section in the cross-machine direction (magnification: 20X) is shown in FIG. 19, and a photomicrograph of a cross-section parallel to the machine direction that passes through the tops of the ridges (magnification: 20X) is shown in FIG. 20.

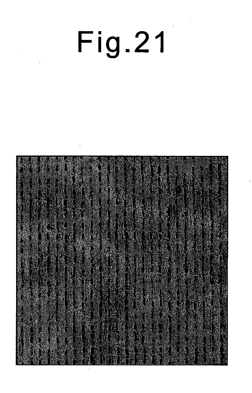

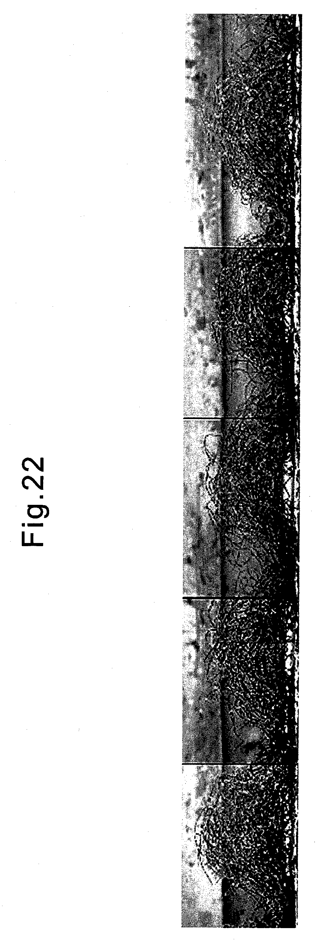

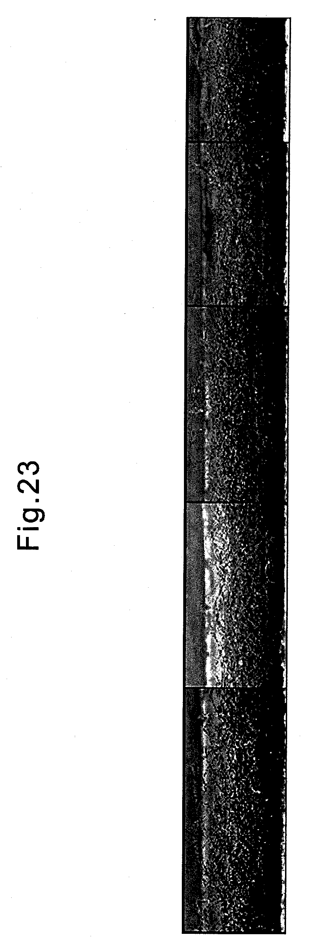

An overhead photograph of the first surface of the non-woven fabric sheet of Comparative Example 1 (digital camera, scanner incorporation, actual size) is shown in FIG. 21, a photomicrograph of a cross-section in the cross-machine direction (magnification: 20X) is shown in FIG. 22, and a photomicrograph of a cross-section parallel to the machine direction that passes through the tops of the ridges (magnification: 20X) is shown in FIG. 23.

The fiber orientation of ridges of the non-woven fabric sheet of Example 3 is shown in FIG. 24. In addition, the fiber orientation of ridges of the non-woven fabric sheet of Comparative Example 1 is shown in FIG. 25.

INDUSTRIAL APPLICABILITY

The non-woven fabric sheet of the present invention can be preferably used as a member of absorbent articles such as disposable diapers, sanitary napkins and cleaning wipes.

BRIEF DESCRIPTION OF THE REFERENCE SYMBOLS

| 1  | Non-woven fabric sheet |
|----|-----------------------|
| 2  | Ridges |
| 3  | Grooves |
| 4  | First fibrous layer |
| 5  | Second fibrous layer |
| 6  | Through holes or thin-walled portions |
| 7  | Bridges |
| 8  | Portions where fiber assembly density is relatively high |
| 9  | Portions where fiber assembly density is relatively low |
| 11 | Container |
| 12 | Carding machine |
| 13 | Web |
| 14 | Container |
| 15 | Carding machine |
| 16 | Web |
| 17 | Endless belt |
| 18 | Laminated web |

| 19 | Suction drum |
|----|----|
| 20 | Nozzles |
| 21,22,23 | Nozzle rows |
| 24 | Web |
| 25 | Endless belt |
| 26 | First heat treatment dryer |
| 27 | Endless belt |
| 28 | Second heat treatment dryer |
| 29 | Endless belt |
| 30 | Endless belt |
| 31 | Roller |
| 41 | Molding plate |
| 42 | Liquid passage portions |
| 43 | Liquid blocking portions |
| 44 | Holes |
| 50 | Floating dryer |
| 51,52 | Mesh conveyors |
| 53 | Hot air outlets |

**Claims**

1. A non-woven fabric sheet in which a plurality of ridges and grooves are formed on a first surface thereof extending in a machine direction, wherein the non-woven fabric sheet comprises a first fibrous layer on a first surface side and a second fibrous layer on a second surface side opposite from the first surface side, the second fibrous layer comprises latent crimpable fibers, the first fibrous layer comprises heat-fusible fibers that do not demonstrate crimping at the crimping starting temperature of the latent crimpable fibers, and when the non-woven fabric sheet is viewed from overhead from the first surface side, the outer edges of the ridges have a repeated meandering shape.

2. The non-woven fabric sheet according to claim 1, wherein, sites where the height of the ridges is relatively high over the direction in which the ridges extend and sites where it is relatively low are formed in the ridges.

3. The non-woven fabric sheet according to claim 1 or 2, wherein the ridges have an enhanced three-dimensional random orientation as a result of at least a portion of the fibers therein generating curvature or bending.

4. A process for producing a non-woven fabric sheet, comprising:

   a) a step for opening a fiber assembly containing latent crimpable fibers by passing through a carding machine to form a web containing latent crimpable fibers,
   b) a step for opening a fiber assembly containing heat-fusible fibers that do not demonstrate crimping at the crimping starting temperature of the latent crimpable fibers by passing through a carding machine to form a web containing heat-fusible fibers,
   c) a step for superimposing the web containing latent crimpable fibers and the web containing heat-fusible fibers to form a laminated web,
   d) a step for realigning the fibers so that ridges and grooves are formed in the laminated web, and portions in which the degree of fiber entanglement in the direction in which the ridges are continuous is relatively high and portions in which it is relatively low are alternately formed,
   f) a step for heating the web in which fibers have been realigned to cause the latent crimpable fibers to demonstrate crimping, and
   g) a step for heating the web in which crimping has been demonstrated by the latent crimpable fibers to fuse the heat-fusible fibers at sites where they mutually intersect.

5. The process according to claim 4, wherein step d is a step in which the laminated web is transported by placing on a support, in which liquid passage portions and liquid blocking portions extending in parallel in a cross-machine direction are arranged by alternately repeating in a machine direction, while forming a plurality of ridges and grooves extending in parallel in the machine direction by spraying a liquid from a plurality of nozzles arranged in a row in the cross-machine direction onto the surface of the web side of the laminated web that contains the heat-fusible fibers.

**6.** The process according to claim 4 or 5, wherein step f is a step in which the web is heated to a temperature at which the latent crimpable fibers demonstrate crimping and which is lower than the fusing temperature of the heat-fusible fibers.

**7.** The process according to any of claims 4 to 6, wherein step f is carried out in a state in which there is little resistance to force that attempts to shrink the latent crimpable fibers in the machine direction as a result of crimping.

**8.** The process according to claim 7, wherein the transport speed of step f is slower than that of the previous step.

**9.** The process according to claim 7 or 8, wherein a floating dryer is used in step f.

**10.** The process according to any of claims 4 to 9, comprising

> e) a step for transporting the laminated web obtained in step d to step f
> between step d and step f.

**11.** The process according to any of claims 4 to 10, comprising

> h) a step for cooling the web obtained in step g
> after step g.

**12.** An absorbent article containing the non-woven fabric sheet according to any of claims 1 to 3.

# Fig.1

# Fig.2

# Fig.3

# Fig.4

Fig.5

# Fig.6

(a)

P

20

20

(b)

21 →
22 →
23 →

P

Q

20

20

CD

MD

# Fig.7

21  22  23

18

24

19

17

25

# Fig.8

Fig.9

# Fig.10

# Fig.11

# Fig.12

(a)

**IMAGE THRESHOLDING** ☒

0    127    255

50   100   150   200

METHOD
- ⊙
- ○
- ○

OK

CANCEL (C)

☑ NEW IMAGE (N)

OTSU
METHOD (O)    DYNAMIC
              THRESHOLDING (A)

STATIC THRESHOLDING (F)

(b)

FEATURE VALUE

LABELED IMAGE

**SHAPE FEATURE EXTRACTION** ☒

COUNT          LIMITED EXTRACTION

EXTRACTION TARGET SETTING
☑ WIDTH RANGE [0]  [640] PIXELS
☑ HEIGHT RANGE [0] [480] PIXELS

TARGET COLOR
⊙ BLACK  ○ WHITE      COUNT

UPDATE (U)

QUIT (Q)

EXTRACTION NUMBER
[16]

(c)

NET AREA        FEATURE VALUE

**SHAPE FEATURE EXTRACTION** ☒

COUNT          LIMITED EXTRACTION

HOLE AREA
TOTAL AREA
HOLE AREA RATIO(%)
OUTER CIRCUMFERENCE

FEATURE
VALUE

EQUIVALENT
CIRCLE DIAMETER

UPDATE (U)

QUIT (Q)

LABELED IMAGE

EXTRACTION NUMBER
[16]

INNER CIRCUMFERENCE

## Fig.13

## Fig.14

## Fig.15

Fig.16

Fig.17

Fig.18

Fig.19

Fig.20

Fig.21

Fig.22

Fig.23

# Fig.24

# Fig.25

<table>
<tr><td colspan="3" align="center">**INTERNATIONAL SEARCH REPORT**</td><td colspan="2">International application No.<br>PCT/JP2011/056321</td></tr>
</table>

| | |
|---|---|
| **A. CLASSIFICATION OF SUBJECT MATTER**<br>*D04H1/70*(2006.01)i, *A61F13/49*(2006.01)i, *A61F13/511*(2006.01)i, *B32B5/26*(2006.01)i, *D04H1/54*(2006.01)i | |

According to International Patent Classification (IPC) or to both national classification and IPC

**B. FIELDS SEARCHED**

Minimum documentation searched (classification system followed by classification symbols)
D04H1/00-18/00, A61F13/49, A61F13/511, B32B1/00-43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2004-169235 A (Kao Corp.),<br>17 June 2004 (17.06.2004),<br>claim 1; paragraphs [0001], [0011] to [0013],<br>[0019], [0022], [0024], [0029], [0032], [0036]<br>(Family: none) | 1,3-6,10-12<br>2,7-9 |
| Y | JP 2008-25081 A (Uni-Charm Corp.),<br>07 February 2008 (07.02.2008),<br>claims 1, 9; paragraphs [0004], [0140] to<br>[0149]; fig. 17, 18<br>& US 2007/0298213 A1<br>claims 1, 9; paragraphs [0008], [0166] to<br>[0175]<br>& EP 2034072 A1        & WO 2007/148497 A1<br>& KR 10-2009-0018059 A   & CN 101443499 A | 2 |

| | | | |
|---|---|---|---|
| ☒ Further documents are listed in the continuation of Box C. | | ☐ See patent family annex. | |

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| | |
|---|---|
| Date of the actual completion of the international search<br>09 May, 2011 (09.05.11) | Date of mailing of the international search report<br>17 May, 2011 (17.05.11) |
| Name and mailing address of the ISA/<br>Japanese Patent Office | Authorized officer |
| Facsimile No. | Telephone No. |

**INTERNATIONAL SEARCH REPORT**

| International application No. |
| --- |
| PCT/JP2011/056321 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | WO 2008/69199 A1  (Uni-Charm Corp.),<br>12 June 2008 (12.06.2008),<br>claim 8; paragraphs [0087], [0088], [0112],<br>[0114]<br>& US 2008/0132136 A1<br>paragraphs [0106], [0107], [0131], [0133]<br>& EP 2090683 A1          & JP 2008-138340 A<br>& KR 10-2009-0086587 A   & CN 101553611 A | 7,8 |
| Y | JP 2001-40531 A  (Chisso Corp.),<br>13 February 2001 (13.02.2001),<br>paragraph [0022]<br>& US 6274237 B1<br>column 6, lines 5 to 19 | 9 |
| A | JP 2008-161302 A  (Kao Corp.),<br>17 July 2008 (17.07.2008),<br>entire text<br>& KR 10-2008-0061283 A   & CN 101219078 A | 1-12 |
| A | JP 2009-160919 A  (Kao Corp.),<br>23 July 2009 (23.07.2009),<br>entire text<br>& US 2010/0234823 A       & EP 2221172 A1<br>& WO 2009/075197 A1       & KR 10-2010-0095572 A | 1-12 |
| A | JP 2010-5925 A  (Kao Corp.),<br>14 January 2010 (14.01.2010),<br>entire text<br>(Family: none) | 1-12 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3617637 B